# EUROPEAN PATENT APPLICATION

(11) **EP 4 212 120 A1**
(43) Date of publication of application: **19.07.2023**
(21) Application number: 22151243.7
(22) Date of filing: 12.01.2022
(51) Int. Cl.: A61B 18/14

(54) **ELECTRICAL ABLATION DEVICE**

(71) Applicant: AtriAN Medical Limited, H91 E79C Galway (IE)
(72) Inventor: DEANE, Stuart, Galway, H91 E79C (IE); REILLY, John, Galway, H91 E79C (IE); COFFEY, Kenneth W., Galway, H91 E79C (IE); O'BRIEN, Barry, Galway, H91 E79C (IE)
(74) Representative: Murgitroyd & Company

(57) **Abstract**

A device for ablating ganglionated plexi neuron cell bodies to treat cardiac disorders comprising a catheter and electrodes with a source of electrical energy to help ablate target tissue.

## Description

This invention relates generally to ablation of tissue in particular to an electrical device for ablating ganglionated plexi neuron cell bodies to treat cardiac disorders, and methods of using such a device.

Many people suffer from abnormal heart conditions. Cardiac arrhythmias, such as atrial fibrillation, occurs when regions of the cardiac tissue abnormally conduct electric signals to adjacent tissue, thereby disrupting the normal cycle and causing asynchronous rhythm.

Procedures for treating arrhythmia include surgically disrupting the origin of the signals causing the arrhythmia, or disrupting the conducting pathway for such signals. By selectively ablating certain cardiac tissue by the application of energy, for example, it may be possible to stop or alter the signals from one portion of the heart to another. The ablation process helps to destroy abnormal signal-making cells or the pathway of the abnormal signal.

The ablation energy required may be conveyed through electrodes, often the energy is passed from one electrode to another electrode, with the energy travelling through the target tissue to be ablated. Catheters are often used to help position the electrode or at least direct the energy used for ablation. However, the procedure is still difficult, and time consuming, and has risks for the patient.

In a first aspect of the present invention there is provided a device for ablating cell bodies to treat cardiac disorders, the device comprising:
- at least one pair of oppositely charged electrodes;
- a source of electrical energy; and
- a catheter, the catheter comprising a proximal end, a distal end and an internal conduit between the proximal end and distal end, and the catheter further comprising at least one opening, and wherein at least one of the oppositely charged electrodes is at least partially housed in the catheter.

In some embodiments the device is for selectively ablating cell bodies comprises selectively ablating ganglionated plexi neuron cell bodies to treat cardiac disorders. In some other embodiments, the device is for selectively ablating cell bodies comprises selectively ablating any tissue or cell bodies associated with the heart, to treat cardiac disorders.

The catheter is typically configured to aid positioning within the body. Typically, the catheter is elongated in shape; and pointed, rounded or smooth, at one end, for example the distal end. In some embodiments, one end, for example, the distal end of the catheter comprises a tapered, or pointed, or acuminate shape. In some embodiments, the catheter comprises an atraumatic shaped end. This helps to enable positioning of the catheter within the patient and to reach a good position for any ablation procedure. Ideally, for epicardial ablations the catheter is configured to be able to be inserted into the pericardial space at an entry point. In some embodiments, the distal end of the catheter is configured for insertion into the pericardial space at an entry point.

In some embodiments, the catheter at least partially houses at least one electrode. The at least one electrode that is at least partially housed within the catheter may be an anode or a cathode. In embodiments where there are more than one electrode, the device may comprise both cathode electrodes and anode electrodes.

In some embodiments the at least one electrode that is partially housed within the catheter is partially housed within the conduit of the catheter. The conduit of the catheter may also, partially, house the wiring required for the at least one electrode partially housed within the catheter. The wiring of the at least one electrode at least partially housed within the conduit may be connected to the energy source. Conveniently, the conduit of the catheter can house the wires of the various electrodes of the device.

The conduit of the catheter also enables any saline used to make contact with the at least one electrode at least partially housed within the catheter. The conduit can also allow fluid communication of any saline solution used, to make contact with any and all the electrodes within the catheter but also assist in enabling saline solution to be dispensed from the catheter and make improved electrical contact with the adjacent or near by tissue, and thus assist in spreading the electric field from the device when used.

In some embodiments of the present invention there further comprises a dispersive pad, wherein the dispersive pad comprises one of the electrodes of the at least one pair of oppositely charged electrodes. The dispersive pad may be configured to be placed a distance from the catheter of the device. For example, the dispersive pad may be placed exterior of the patient. The dispersive pad used in some examples may be place on the skin of the patient on the back of the patient or in other examples of use on the chest of the patient. Ideally, the dispersive pad in embodiments where a dispersive pad is used is placed such that the target tissue to be ablated is between the electrode facing side of the catheter and the dispersive pad.

The at least one pair of electrodes can be any known electrode suitable for conveying energy within the body of the patient. Ideally, the, or at least one electrode is at least partially housed within the catheter. The electrodes may comprise platinum iridium or stainless steel for example.

In some embodiments, the catheter comprises a plurality of electrodes. In some embodiments, the catheter comprises a plurality of positive electrodes. In some embodiments, the catheter comprises a plurality of negative electrodes. In some embodiments, the catheter comprises a plurality of electrodes, both positive and negative electrodes. In some embodiments, the catheter comprises a row of alternating positive and negative electrodes.

In some embodiments, the catheter comprises a plurality of anode electrodes. In some embodiments, all of the electrodes in the catheter are anodes. In some embodiments, the catheter comprises all cathode electrodes.

In some embodiments the catheter comprises a plurality of both cathode and anode electrodes. In specific embodiments, the catheter comprises a plurality of both anode and cathode electrodes, wherein the electrodes alternate between anode and cathode electrodes along the longitudinal length of the catheter.

In some alternative embodiments, the catheter comprises a plurality of both cathode and anode electrodes, wherein the electrodes alternate in pairs of cathode and anode electrodes along the longitudinal length of the catheter.

In some embodiments the number of electrodes is an uneven number. In some embodiments the number of anode electrodes does not equal the number of cathode electrodes.

In some embodiments the outer radially facing surface of the electrodes are between 2 and 4 millimetres in length and between 1.5 and 2.5 millimetres in width. In specific embodiments the electrodes comprise an outer diameter (OD) of 2.2mm, an inner diameter (ID) of 1.9mm a length, longitudinal axis wise, of 3.18mm. In alternative specific embodiments the electrodes comprise an outer diameter (OD) of 3.759 mm, inner diameter (ID) of 3.429 mm, and a length of 3.18mm. In some embodiments the diameter of the catheter is approximately 8.5 French. In some specific embodiments the radially facing surface of the electrodes are 3.2 by 2.5 millimetres. In some alternative embodiments the radially facing surface of the electrodes are 2.7 by 2 millimetres.

In some embodiments, the catheter comprises an opening. In some embodiments the catheter comprises an opening, wherein the opening is positioned at, or near to, one end of the catheter, for example the distal end. In some embodiments the opening is configured to expose a portion of the at least one electrode, for example, to the outside of the catheter. In some embodiments, the opening of the catheter is half the distance of the circumference of the catheter across the longitudinal axis. In other embodiments, the opening is less than half the distance, or less than 180 degrees, of the circumference of the catheter across the longitudinal axis. In some embodiments, the opening is between a quarter and a half of the circumference (distance) of the catheter, or between 90 and 180 degrees, of the circumference of the catheter, across the longitudinal axis. In some embodiments, the opening is between an eighth and a half of the circumference (distance) of the catheter, or between 45 and 180 degrees, of the circumference of the catheter, across the longitudinal axis. In some embodiments, the opening is between an eighth and a quarter of the circumference (distance) of the catheter, or between 45 and 90 degrees of the circumference of the catheter, across the longitudinal axis. In some embodiments, the opening is between 0.5 and 5 millimetres in length across the longitudinal axis direction of the catheter. In specific embodiments, the opening is between 1 and 3 millimetres in length across the longitudinal axis direction of the catheter. In specific embodiments, the opening is 2 millimetres in length across the longitudinal axis direction of the catheter. In some embodiments, the opening is between 2 and 3 millimetres in length along the longitudinal axis direction of the catheter. In specific embodiments, the opening is between 2.5 and 2.9 millimetres in length along the longitudinal axis direction of the catheter. In specific embodiments, the opening is 2.7 millimetres in length along the longitudinal axis direction of the catheter. The opening allows directional targeting of the electric field. The opening assists in enabling the electric field direction to be determined, and thus assist in targeting particular cells or tissue with the electric field, for ablation.

In some embodiments the catheter comprises a shaft wall. The shaft wall may comprise of a polymer, for example PEBAX, a Trade Name for a polymer. The shaft wall of the catheter may comprise a window or opening. The frame or casement of the window or opening may, in some embodiments, be raised radially from the longitudinal axis of the catheter from the wall of the main shaft of the catheter. The frame or casement of the window or opening of the catheter may comprise a radially projecting distance from the longitudinal axis of the catheter that is between 0.1 and 1.2 millimetre greater than the radially projecting distance of the wall of the main shaft of the catheter from the longitudinal axis of the catheter. The frame of the window or opening of the catheter may comprise a radially projecting distance from the longitudinal axis of the catheter that is between 0.1 and 0.6 millimetre greater than the radially projecting distance of the wall of the main shaft of the catheter from the longitudinal axis of the catheter. Having the frame or casement of the window or opening raised from the wall of the catheter enables the electrode to sit proud of the wall of the shaft of the catheter and thus enables a better contact of the catheter with the tissue. As the tissue is soft the electrode protruding from the catheter will push slightly into the tissue giving a good contact, a good electrical connecting contact, that the electric field will pass into the tissue.

In some embodiments, the opening of the catheter is configured to enable an electrode to protrude through the opening of the catheter. In some embodiments, an electrode protrudes through the opening of the catheter. In some embodiments, the at least one oppositely charged electrode partially housed within the catheter protrudes through the opening of the catheter. In some embodiments, an electrode protrudes through the opening from the internal conduit of the catheter. In some embodiments, the opening comprises an electrode wherein the electrode protrudes radially from the longitudinal axis of the catheter by a greater distance than the wall of the catheter. Where the electrode protrudes beyond the wall of the shaft of the catheter in a radially direction this enables the electrode to easily make good contact with the tissue, the electrode is not being blocked by the wall of the catheter. The tissue is soft and thus the electrode protruding beyond the wall of the shaft of the catheter may push into the tissue giving a good contact. In some embodiments the electrode may protrude external of the catheter.

In some embodiments, the opening of the catheter is raised from the main body portion. In some embodiments, the opening of the catheter comprises a radially raised portion, that is radially raised in direction from the longitudinal axis of the catheter. In some embodiments the raised portion, or radially raised portion may be 360 degrees around the circumference of the longitudinal axis of the catheter. In alternative embodiments, the raised portion or radially raised portion of the opening of the catheter may be raised less than 360 degrees around the circumference of the longitudinal axis of the catheter, for example between 45 and 180 degrees around the circumference of the longitudinal axis of the catheter.

In some embodiments at least one electrode of the oppositely charged electrodes that is at least partially housed in the catheter, protrudes external of the catheter beyond the wall of the catheter shaft. In specific embodiments at least one electrode of the oppositely charged electrodes that is at least partially housed in the catheter, protrudes external of the catheter beyond the wall of the catheter shaft in the range of 0.1 to 0.5 millimetres; or 0.1 to 2 millimetres for example. Where the electrode protrudes beyond the wall of the shaft of the catheter in a radially direction this enables the electrode to easily make good contact with the tissue, the electrode is not being blocked by the wall of shaft of the catheter.

In specific embodiments the catheter comprises at least, two raised portions. In specific embodiments, the at least two raised portions are positioned on the catheter wall either side of an opening of the catheter. In some embodiments, the raised portion of the catheter is positioned surrounding an opening of the catheter. In some embodiments the raised portion of the catheter is positioned surrounding an opening of the catheter and is 360 degrees around the circumference of the longitudinal axis of the catheter, apart from at the opening of the catheter.

In some embodiments, the raised portion may be radially raised from the longitudinal axis by 0.1 to 2 millimetres further, than the shaft of the catheter. In some embodiments, the raised portion may be radially raised from the longitudinal axis by 1 millimetre further than the usual wall of the catheter. In some embodiments, the raised portion may protrude between 0.1 to 2 millimetres from the wall of the shaft of the catheter. In some embodiments, the raised portion may protrude by 1 millimetre from the wall of the catheter.

In some embodiments, the raised portion of the catheter comprises an opening. In some embodiments, the opening of the catheter is surrounded by a raised portion.

The raised portions with an opening assist in enabling the electrode to protrude beyond the wall surface of the shaft of the catheter and thus enable tissue within the body to have a good contact with the exposed portion of at least one electrode or plurality of electrodes, partially housed with the catheter.

The electrical energy source may be any electrical energy source able to convey energy to the cells. Typically, the ultimate electrical energy source is mains electricity or a generator producing electricity. In some embodiments, the electrical energy source comprises an output voltage in the range of 500 to 1500 Volts. In some embodiments, the electrical energy source comprises an output voltage in the range of 750 to 1250 Volts. In some embodiments, the electrical energy source comprises an output voltage greater than 900 Volts. In some embodiments, the electrical energy source comprises an output voltage greater than 950 Volts. In specific embodiments, the electrical energy source comprises an output voltage of 1000 Volts.

In some embodiments, the electric field strength at the targeted tissue comprises a voltage field strength in the range of 500 to 1500 Volts/ cm. In some specific embodiments, the electrical energy field at the target tissue comprises a voltage in the range of 750 to 1250 Volts/cm. In some specific embodiments, the electrical field strength at the target tissue comprises a voltage field strength greater than 1000 Volts/cm. In some specific embodiments, the electric field strength at non-targeted cells or tissue is less than 1000 volts/ cm. It is possible in some embodiments, to have the combined features, of an electric field strength greater than 1000 volts/ cm at the target cells and less than 1000 volts/cm at the non-targeted cells or tissue. Advantageously this enables ablation of the targeted cells and tissue and less hard to the non-targeted cells or tissue.

In some embodiments, the energy source is DC voltage. In alternative embodiments, the energy source is AC voltage.

In some embodiments, the electrical source comprises pulsed electricity. In some embodiments, the electrical source comprises a pulsed electric field.

In some embodiments, the pulsed electric field has an output electric field strength greater than 1000 volts per centimetre (V/cm). In some embodiments, the pulsed electric field has an output electric field strength between 1000 volts per centimetre (V/cm) and 1250 volts per centimetre (V/cm). In some embodiments, the pulsed electric field strength has an electric field strength greater than 1000 V/cm at the target cell bodies and transitions to less than 1000 volts per centimetre (V/cm) in the non-targeted cardiac tissue. In some embodiments, the pulsed electric field strength has an electric field strength greater than 1000 V/cm at the ganglionated plexi and transitions to less than 1000 volts per centimetre (V/cm) in the cardiac tissue.

In specific embodiments, the pulsed electric field comprises monophasic pulses. In specific embodiments, the pulsed electric field comprises biphasic pulses.

In some embodiments, the pulsed electric field comprises at least 60 pulses per treatment site, for example, monophasic pulses. In some embodiments, the pulsed electric field comprises at least 90 pulses per treatment site, for example monophasic pulses. In some embodiments, the pulsed electric field comprises between 60 pulses and 120 pulses per treatment site, for example, monophasic pulses. In some embodiments, the pulsed electric field comprises between 60 pulses and 120 pulses per treatment site.

In specific embodiments, the pulse of the electric field comprises a pulse duration of 100 µs. In some embodiments, the pulse of the electric field comprises a pulse duration between 30 and 200 µs. In some embodiments, the pulse of the electric field comprises a pulse duration between 90 and 110 µs.

In some embodiments, the pulse frequency is in the range of 30 to 200 pulses per minute (BPM). In some embodiments, the pulse frequency is in the range of 60 to 120 pulses per minute (BPM). In some embodiments, the pulse delivery is in the range of 0.3 to 3 Hertz (Hz). In alternative embodiments, the pulse frequency is in the range of 0.5 to 1 Hertz (Hz). In specific embodiments, the pulsed delivery is synchronised with the Ventricular Refractory period of the heart of the patient. The Ventricular Refractory period of the heart of a patient can be easily determined by means known to those skilled in the art. In some embodiments the pulse interval is in the range of 0.1 to 1 seconds. The pulse interval is the space between one pulse ending and the next pulse starting.

In some embodiments the at least one electrode comprises at least one port. The at least one port is configured for fluid dispensing and extraction. The port may enable the flow, or positioning, of saline in the desired targeted direction or position. The port may enable the flow of saline from within the catheter, for example, in the conduit of the catheter, or from a source with an outlet within the conduit of the catheter, to an environment outside of the catheter. The port may enable saline to be in contact with the at least one electrode but also assist in directing the electric field from the electrode. In specific embodiments the at least one port of the electrode is positioned to correspond with the opening of the catheter.

In some embodiments, the at least one port of the electrode comprises a diameter in the range of 0.5 to 1.0 millimetres. In some embodiments, the at least one port of the electrode comprises a diameter in the range of 0.25 to 1.5 millimetres. In some embodiments, the at least one port of the electrode comprises a diameter in the range of 0.75 to 1.0 millimetres.

In some embodiments the at least one port of the electrode corresponds in position to at least part of the opening of the catheter. When the port of the electrode and opening of the catheter corresponds, this makes it easy for any liquid, for example saline, to exit the device, thus aids targeting the saline and thus aids targeting the electric field.

In some embodiments, the catheter comprises insulation material. In some embodiments, the catheter comprises an insulation layer. In specific embodiments, the catheter comprises an insulation layer at, or towards, the back of the catheter. The back of the catheter is the non-targeted direction, or portion not comprising an opening, or portion opposite the opening. In some embodiments, the catheter comprises an insulation layer surrounding the openings of the catheter. In some embodiments, the wall of the catheter comprise an insulating layer.

In some embodiments the insulation material comprises a polymer. In some embodiments the insulation material comprises PEBAX, a Trade Name for a polymer.

In some embodiments, the catheter is configured so that in use the catheter may be positioned so that the insulating layer is positioned between the electrode partially housed by the catheter, and the parietal pericardium of the patient. In specific embodiments, the catheter is configured so that in use the opening of the catheter and insulating layer are orientated, configured or positioned, so that the insulating layer can be positioned against the parietal pericardium, or in the direction of the parietal pericardium, and the opening of the catheter can be positioned, configured or orientated against, or towards or facing, the epicardium.

In some embodiments, the catheter comprises an orientation marker. An orientation marker enables a user to confirm orientation during use, for example, the radial orientation can be determined via an orientation marker. The orientation marker may simply be an identification mark or sign that identifies a position of the catheter. In some embodiments, the orientation mark is a visible line partially along the longitudinal length on the shaft of the catheter. Ideally, at the proximal end as the proximal end is more likely to be visible when in use. In some embodiments, the orientation mark indicates the orientation of the facing electrodes of the catheter. In some embodiments the orientation mark is a line partially along the circumference of the wall of the shaft of the catheter. In some embodiments the orientation mark comprises a radiopaque material.

In general, the device of the present invention will have the catheter comprising at least one electrode configured to be positioned near to, or adjacent to the heart of the patient. Techniques are known, for insertion of medical devices within the body, and these may or may not include positioning devices. The catheter may comprise both the anode and cathode electrodes and thus the electric field will mainly be between the electrodes of the catheter, and the near surrounding area. In some methods of using the device, the catheter will comprise at least one electrode, a cathode or anode, and then another oppositely charged electrode will be positioned on the skin of the patient in a desired position so as to target the electric field through the targeted tissue or cell bodies, for example the ganglionated plexi neuron cell bodies of the patient. In some further embodiments the catheter contains both an anode and cathode electrodes adjacent to the heart, without the need for an electrode positioned on the skin.

Typically, the distal end of the catheter will be inserted via an anterior entry point to the pericardial space of the heart of a patient. A portion of the catheter may contact the epicardial surface of the heart. In addition or alternatively, the exposed portion of the electrode may be positioned to face the epicardium of the heart of the patient. When in the desired position, the electrodes may be switched on and the electrode or electrodes activated, to produce the desired electric field, in the desired direction. Tissue, cells and cell bodies in the immediate area of the electric field will receive a higher energy level than tissue or cells further away from the electric field. In addition, tissue or cells in the targeted direction of the electric field will receive a greater energy level than tissue or cells not in the targeted direction and area of the electric field. The device may be repositioned as desired to target other tissue or cells, but also to concentrate the energy on the desired targeted tissue and cells while reducing the energy or period of exposure of the energy of non-targeted tissue or cells. In addition, saline may be dispensed from the catheter, for example from the conduit of the catheter, through the port of the electrode to target the electric energy and electric field to the targeted area and cell bodies. The saline may assist in concentrating the electrical energy or electric field strength in the desired targeted area and to the desired targeted cell bodies, for example ganglionated plexi neuron cell bodies. On completion of the procedure, the catheter and other components of the device are removed from the patient and the insertion holes sealed.

Cells and tissue, for example, the ganglionated plexi neuron cell bodies, that receive a higher electric field strength or a longer exposure to an electric field strength, or a combination of higher field strength and longer duration of electric field, are more likely to be ablated or destroyed.

In another aspect of the present invention there is provided a system for ablating cell bodies for example, ganglionated plexi neuron cell bodies to treat cardiac disorders, the system comprising:
- at least one pair of oppositely charged electrodes;
- a source of electrical energy; and
- a catheter, the catheter comprising a proximal end, a distal end and an internal conduit between the proximal end and distal end, and the catheter further comprising at least one opening, and wherein at least one of the oppositely charged electrodes is at least partially housed in the catheter.

In some embodiments the system of the invention may be for selectively ablating cell bodies.

In some embodiments, the system comprises a device for ablating cell bodies, for example, ganglionated plexi neuron cell bodies to treat cardiac disorders, as described herein to the first aspect of the present invention. In some embodiments, the system comprises a device for selectively ablating cell bodies, for example, ganglionated plexi neuron cell bodies to treat cardiac disorders, as described herein to the first aspect of the present invention.

In some embodiments of the system, the system further comprises a saline solution. In some embodiments of the system, the system further comprises a saline solution source. In some embodiments of the system, the system further comprises a saline solution source that comprises an outlet within the conduit of the catheter. In some embodiments of the system, the system further comprises a control for varying the amount, and flow rate of the saline from the catheter.

The saline solution acts to conduct the electric field in the area of the saline solution. Saline may assist in targeting the electric field to the desired location. The saline solution may assist in isolating the electric field, in that the electric field may be strong adjacent to or within the area of the saline solution but weaker when not adjacent to or within the area of the saline solution. The saline solution may assist in spreading the electric field in the desired direction. This is advantageous as the saline may reach tissue that the catheter cannot easily make contact with.

In some embodiments, the saline solution concentration is 0.9% concentration of salt in water. Although alternative concentrations may be used in other embodiments.

In some embodiments, the electric field strength at, or of, the saline solution comprises an electric field strength in the range of 500 to 1500 Volts/ cm. In some specific embodiments, the electrical field strength at, or of, the saline solution comprises a voltage in the range of 750 to 1250 Volts/cm.

In some system embodiments of the present invention there comprises a dispersive pad, wherein the dispersive pad comprises one of the electrodes of the at least one pair of oppositely charged electrodes. In some embodiments, the dispersive pad comprises an anode electrode. In alternative embodiments, the dispersive pad comprises a cathode electrode. The dispersive pad may be configured to be placed external of the patient, for example on the skin of the back or chest of the patient.

In some system embodiments of the present invention, there further comprises a delivery system for deploying the catheter. The delivery system may assist in deploying the catheter in a desired position within the patient, for example, but not limited to; within a blood vessel; a chamber of the body; or within the pericardial space of a patient.

In a further aspect of the present invention there is provided a method of ablating cell bodies, for example ganglionated plexi neuron cell bodies, to treat cardiac disorders comprising the steps of:
- delivering the electric field between at least two oppositely charged electrode
- providing a source of an electric field;
- providing a catheter having a proximal end, a distal end, and an internal conduit between the proximal end and distal end, wherein the catheter at least partially houses at least one of the two oppositely charged electrodes and the catheter further comprises, at or near the distal tip at least one opening;
- inserting the catheter via an anterior entry point, of a patient, to the pericardial space of the patient;
- positioning the catheter within the pericardial space; and
- applying the electric field to the targeted tissue.

In some embodiments, the method further comprises the step of selectively ablating cell bodies for example, ganglionated plexi neurons cell bodies to treat cardiac disorders. In some embodiments, the method further comprises the step of ablating ganglionated plexi neurons cell bodies to treat cardiac disorders.

In some embodiments the method of ablating cell bodies, for example, ganglionated plexi neuron cell bodies to treat cardiac disorders may comprise the steps of using the device of the first aspect of the present invention as described herein, or using the system of the present invention as described herein.

In some method embodiments of the present invention, there further comprises the step of positioning the catheter at a desired location by the use of a delivery system for deploying the catheter and deploying the catheter at the desired position within the patient. The delivery system may assist in deploying the catheter in a desired position within the patient; for example but not limited to, within a blood vessel; a chamber of the body; or within the pericardial space of a patient.

In some method embodiments of the present invention there further comprises the step of delivering an electric field sufficient enough to ablate the desired target cells or tissue. In some method embodiments of the present invention there further comprises the step of delivering an electric field sufficient enough to achieve an electric field strength of greater than 900 or 1000 Volts per centimetre.

In some embodiments, the targeted tissue for ablating comprises ganglionated plexi neuron cell bodies. In some embodiments, the method further comprises the step of applying the electric field to the targeted tissue comprising ganglionated plexi neuron cell bodies.

In specific embodiments, the applied electric field is of sufficient strength to ablate the targeted tissue, for example, to ablate the ganglionated plexi neuron cell bodies. In some embodiments, the method further comprises the step of applying sufficient strength of the electric field to effectively ablate the targeted tissue, for example, ganglionated plexi neuron cell bodies.

In some embodiments, the method comprises the step of delivering a pulsed electric field that has an output electric field strength greater than 1000 volts per centimetre (V/cm). In some embodiments, the method further comprises the step of delivering a pulsed electric field that has an output electric field strength between 1000 volts per centimetre (V/cm) and 1250 volts per centimetre (V/cm).

In some method embodiments of the present invention there further comprises the step of delivering an electric field sufficient enough to ablate the desired target cells or tissue. In some method embodiments of the present invention there further comprises the step of delivering an electric field sufficient enough to achieve an electric field strength of greater than 900 or 1000 Volts per centimetre. In some embodiments, the method further comprises the step of delivering a pulsed electric field strength that has an electric field strength greater than 1000 V/cm at the target cell bodies. In some embodiments, the method further comprises the step of delivering a pulsed electric field strength that has an electric field strength greater than 1000 V/cm at the target cell bodies and transitions to less than 1000 volts per centimetre (V/cm) in the non-targeted cardiac tissue. In some embodiments, the method further comprises the step of delivering a pulsed electric field strength that has an electric field strength greater than 1000 V/cm at the ganglionated plexi and transitions to less than 1000 volts per centimetre (V/cm) in the cardiac tissue.

In specific embodiments the duration of the applied electric field is of sufficient time or duration to effectively ablate the targeted tissue, for example to ablate the ganglionated plexi neuron cell bodies. In some embodiments, the method further comprises the step of applying the electric field energy of sufficient time or duration to ablate the targeted tissue, for example, ganglionated plexi neuron cell bodies.

In specific embodiments, the direction of the applied electric field is of sufficient direction to effectively ablate the targeted tissue, for example to ablate the ganglionated plexi neuron cell bodies. In some embodiments, the method further comprises the step of applying the electric field energy of sufficient direction to ablate the targeted tissue, for example, ganglionated plexi neuron cell bodies

In specific embodiments the direction, duration and strength of the applied electric field is sufficient to ablate the targeted tissue, for example to ablate the ganglionated plexi neuron cell bodies. In some embodiments, the method further comprises the step of applying the electric field energy of sufficient duration, direction and strength to ablate the targeted tissue, for example, to ablate targeted ganglionated plexi neuron cell bodies.

In some embodiments, the method further comprises the step of applying a pulsed electric field that comprises at least 60 pulses per treatment site, for example, monophasic pulses. In some embodiments, the method further comprises the step of applying a pulsed electric field that comprises at least 90 pulses per treatment site, for example, monophasic pulses. In some embodiments, the method further comprises the step of applying a pulsed electric field that comprises between 60 pulses and 120 pulses per treatment site, for example, monophasic pulses. In some embodiments, the pulsed electric field comprises between 60 pulses and 120 pulses per minute.

In specific embodiments, the method further comprises the step of applying a pulse of the electric field that comprises a pulse duration of 100 µs. In some embodiments, the method further comprises the step of applying a pulse of the electric field that comprises a pulse direction between 90 and 110 µs.

In some embodiments the electric field, or pulsed electric field, is effective to ablate the targeted tissue, for example ganglionated plexi neuron cell bodies. In some embodiments the method further comprises the step of applying the electric field, or pulsed electric field, sufficiently in direction, duration or strength or any combination of direction, duration or strength to effectively ablate the target tissue or cells, for example the ganglionated plexi neuron cell bodies.

In some embodiments the step of providing a source of an electric field comprising providing a source of a pulsed electric field.

In some embodiments the method further comprises providing a saline solution to be in contact with the at least one of the two oppositely charged electrodes that is at least partially housed with the catheter.

In some embodiments the method further comprises providing a saline solution to be in contact with, or positioned to be near to, the targeted tissue for ablating for example ganglionated plexi neuron cell bodies.

In some embodiments, the method of ablating cell bodies, for example ganglionated plexi neuron cell bodies further comprises the step of: positioning the catheter, wherein the catheter comprises an insulating layer, so that in use the catheter is positioned so that the insulating layer is positioned between the electrode partially housed by the catheter, and the parietal pericardium of the patient.

In some embodiments, the method of ablating cell bodies, for example ganglionated plexi neuron cell bodies, further comprises the step of: positioning the catheter, wherein the catheter comprises an opening that is configured to expose at least part of the electrode, so that in use the catheter is positioned so that the electrode is adjacent to, or near to, or in the direction of the epicardium.

In some embodiments the electric field, or pulsed electric field, is effective to ablate autonomic nervous system activity. In some embodiments, the method further comprises the step of applying the electric field sufficiently in direction, duration or strength or any combination of direction, duration or strength to effectively ablate autonomic nervous system activity.

In some embodiments the catheter is positioned such that the at least one of the two oppositely charged electrodes, that is at least partially housed within the catheter, is at least partially exposed to an outside of the catheter at the at least one opening of the catheter.

In some embodiments, the method further comprises the step of applying the pulsed electric field wherein the pulsed electric field provides an electric field strength greater than 1000 volts per centimetre (V/cm). In some embodiments, the method further comprises the step of applying the pulsed electric field wherein the pulsed electric field provides an electric field strength between 1000 volts per centimetre (V/cm) and 10,000 volts per centimetre (V/cm). In some embodiments, the method further comprises the step of applying the pulsed electric field wherein the pulsed electric field provides an electric field strength between 1000 volts per centimetre (V/cm) and 1250 volts per centimetre (V/cm).

In some specific embodiments, the method further comprises the step of applying the pulsed electric field wherein the pulsed electric field provides an electric field strength greater than 1000 volts per centimetre (V/cm) at the targeted cell bodies, for example, ganglionated plexi tissue and transitions to less than 1000 volts per centimetre (V/cm) within non-targeted tissue, for example, the cardiac tissue.

In some specific embodiments, the method further comprises the step of applying the pulsed electric field wherein the pulsed electric field provides an electric field strength greater than 1000 volts per centimetre (V/cm) adjacent to the exposed portion of the electrode or saline solution. In some specific embodiments, the method further comprises the step of applying the pulsed electric field wherein the pulsed electric field provides an electric field strength greater than 1000 volts per centimetre (V/cm) adjacent to the exposed portion of the electrode or saline solution and transitions to less than 1000 volts per centimetre (V/cm) within the cardiac tissue or a further distance than 1 centimetre from the catheter or saline solution.

In some specific embodiments, the method further comprises the step of applying the pulsed electric field wherein the pulsed electric field provides an electric field strength greater than 1000 volts per centimetre (V/cm) adjacent to, or near to, the exposed portion of the electrode or saline solution or within 1 centimetre of the catheter or within 1 centimetre of the saline solution. In some specific embodiments, the method further comprises the step of applying the pulsed electric field wherein the pulsed electric field provides an electric field strength greater than 1000 volts per centimetre (V/cm) adjacent to, or near to, the exposed portion of the electrode or saline solution or within 1 centimetre of the exposed portion of the electrode or within 1 centimetre of the saline solution; and transitions to less than 1000 volts per centimetre (V/cm) within the cardiac tissue or further from the exposed portion of the electrode or saline solution than 1 centimetre.

In some embodiments, the method further comprising the step of providing the pulsed electric field wherein the pulsed electric field provides at least 10 repeat monophasic pulses per pulse sequence In some embodiments, the method further comprising the step of providing the pulsed electric field wherein the pulsed electric field provides at least 60 repeat monophasic pulses per pulse sequence. In specific embodiments, the method further comprising the step of providing the pulsed electric field wherein the pulsed electric field provides at least 50 repeat monophasic pulses per pulse sequence. In specific embodiments, the method further comprising the step of providing the pulsed electric field wherein the pulsed electric field provides at least 90 repeat monophasic pulses per pulse sequence. In specific embodiments, the method further comprising the step of providing the pulsed electric field wherein the pulsed electric field provides at least 100 repeat monophasic pulses per pulse sequence. In specific embodiments, the method further comprising the step of providing the pulsed electric field wherein the pulsed electric field provides at least 110 repeat monophasic pulses per pulse sequence. In specific embodiments, the method further comprising the step of providing the pulsed electric field wherein the pulsed electric field provides between 10 and 150 repeat monophasic pulses per pulse sequence.

In some embodiments, there is an interval between pulse sequences and there may be a plurality of pulse sequences per treatment site. In some embodiments, the method comprises the step of repeating pulse sequences per treatment site. In specific embodiments the method comprises the step of providing 10 pulses per pulse sequence and after an interval repeating the pulse sequence a plurality of times, for example 6 times, per treatment site.

In some embodiments, the method further comprises the step of providing the pulsed electric field having a pulse duration of 100 microsecond (µs) for each monophasic pulse. In some embodiments, the method further comprises the step of providing the pulsed electric field having a pulse duration between 80 and 110 microseconds per monophasic pulse.

In some embodiments, the method further comprising the step of providing the pulsed electric field wherein the pulsed electric field provides at least 60 repeat monophasic pulses having a pulse duration of 100 microseconds (µs).

In some embodiments, the method further comprises the step of providing the pulsed electric field having a pulse interval in the range of 0.1 to 10 seconds. In some embodiments, the method comprises a pulse frequency that is synchronised with the Ventricular Refractory period of the heart of the patient. In some embodiments, the method further comprises the step of synchronising the pulse interval to the ventricular refractory period of the patient. In some embodiments, the method further comprises the delivering a pulsed electric field comprising a pulse interval of 0.5 to 1 seconds.

In some embodiments the method further comprising the step of irrigating saline solution through the at least one port via the internal conduit at a flow rate in the range of 0.5 to 20 millilitres per minute (ml/min). In specific embodiments, the method further comprises the step of irrigating saline solution at a flow rate in the range of 1 to 10 millilitres per minute (ml/min). In some embodiments, the method further comprises providing a saline solution. In some embodiments, the method further comprises providing a source of saline solution.

In some embodiments the method further comprises the step of positioning the at least one electrode which is partially housed in the catheter, such that the distance between the target tissue, for example, neuron cell bodies, and the at least one electrode is in the range of 0.1-10 millimetres.

In some embodiments in use the catheter of the device as described herein is positioned in the body in proximity with the target tissue and cell bodies to be ablated, for example, ganglionated plexi neuron cell bodies. A desired amount of saline solution may be dispensed from, for example, a saline source, or outlet, within the conduit 4 of the catheter. In this some embodiments 42 millilitres of saline may be dispensed, although in other embodiments other amounts of saline solution may be dispensed. In some embodiments, the saline may dispensed at 1 millilitre per second. The electrode may be activated and an electric field may be produced around the exposed portion of the electrode. The voltage output of the electrode may be 1000 volts DC. The electric field may be a pulsed electric field. In some embodiments, the electric field may comprise 60 monophasic electric pulses of a duration of 100 microseconds at a pulse frequency of 0.3 to 3 Hertz (Hz).

By the term "ablating" or ablation" or the like as used herein, these terms include removal, destruction or killing of a cell or tissue.

By the term, "cell body" or "cell bodies" as used herein this term is used to describe tissue or tissues; or a cell or cells, of the body, for example, of the body of the patient.

By the term "cm" as used herein this term is used to mean centimetre or centimetres.

By the term "distal end" as used herein, this term is used to describe the further end for example the end that may lead positioning within the body. However, the term is used to aid understanding and is not necessarily limiting and could be used interchangeably with "proximal end".

By the term "electric field" as used herein this is used to describe the region around the charged element, for example, the electrode that within which a force would be exerted on any other charged elements of objects, and is defined as the electric force in voltage per unit rate of change for example volts per centimetre (V/cm)

By the term, "exposed" as used herein this term is used to mean not covered. For example, the exposed portion of the electrode is a portion not covered completely by the catheter wall or walls due to the opening of the catheter.

By the term "frame" this is used interchangeably with the word "casement" and these terms are used to describe an item that at least partially surrounds another item, or space.

By the term "mm" as used herein this is used to mean millimetre or millimetres.

By the term "ml" as used herein, this term is used to mean millimetre or millilitre

By the term "oppositely charged electrodes" as used herein, this is used to mean at least one electrode that is positively charged and at least one electrode that is negatively charged. Or able to be so charged. The term includes negative and positive electrodes.

By the term "opening" as used herein, this term is used interchangeably with "window" and is used to describe an aperture, or hole or the like.

By the term "exposed portion of the electrode" as used herein this is used to describe the portion of the electrode, that is partially housed within the catheter, but is not a portion covered by the catheter or covered by the insulating material or insulating layer. The exposed portion of the electrode is a position of the electrode that can make contact with a saline solution when used, and may be able to make contact with tissue within the body.

By the term "near to" or "positioned near to" as used herein this is used to mean within 10 millimetres.

By the term "protrudes" as used herein, this term is used to describe an item that extends beyond another, for example where the electrode protrudes through the opening of the wall of the shaft of the catheter the furthest part of the electrode from the axis of the catheter, is a further distance from the axis of the catheter, than the wall of the shaft of the catheter.

By the term "proximal end" as used herein, this term is used to describe the nearer end for example the end that may be closest to the user positioning the device. However, the term is used to aid understanding and is not necessarily limiting and could be used interchangeably with "distal end".

By the term, "pulse frequency" this is used to describe the number of pulses per second, for example of pulsed energy.

By the term "radiopaque" as used herein, is used to describe an item that is opaque to X-rays or similar radiation.

By the term, "saline" or "saline solution" as used herein, these terms are used interchangeably, and these terms describe a saline solution that allows electricity to be conducted along the saline solution.

By the term "selectively" as used herein, this term is used to describe that some , not necessarily all, particularly items are selected for example some desired targeted cells are selected for ablation.

By the term "shaft" as used herein this term is used to describe the elongated often cylindrical part of a catheter.

By the term "ventricular refractory period" (VRP) as used herein this term is used to describe the Effective Refractory Period (ERP) of the ventricular tissue, which is the period of time in the cardiac cycle, after an action potential is initiated, that a new action potential cannot be initiated.

By the term "wall of the shaft of the catheter" as used herein this term is used to describe the main wall of the shaft along the length of the catheter, not any raised portions from this wall.

Any feature or combination of features of any example, embodiment or aspect, of the invention may be used or combined with any other feature or combination of features of any example, embodiment or aspect of the invention.

Non-limiting examples will be further described in relation to the figures:
Figure 1 shows part of a catheter according to the present invention;
Figure 2 shows part of another catheter according to the present invention;
Figure 3 shows part of another catheter according to the present invention;
Figure 4 shows part of another catheter according to the present invention;
Figure 5A shows part of another catheter according to the present invention;
Figure 5B shows part of another catheter according to the present invention;
Figure 5C shows part of another catheter according to the present invention;
Figure 6 shows part of another catheter according to the present invention;
Figures 7A and 7B show the electric field in different examples;
Figure 8 shows part of a system according to the present invention;
Figure 9 shows an electrode suitable for use with the present invention.

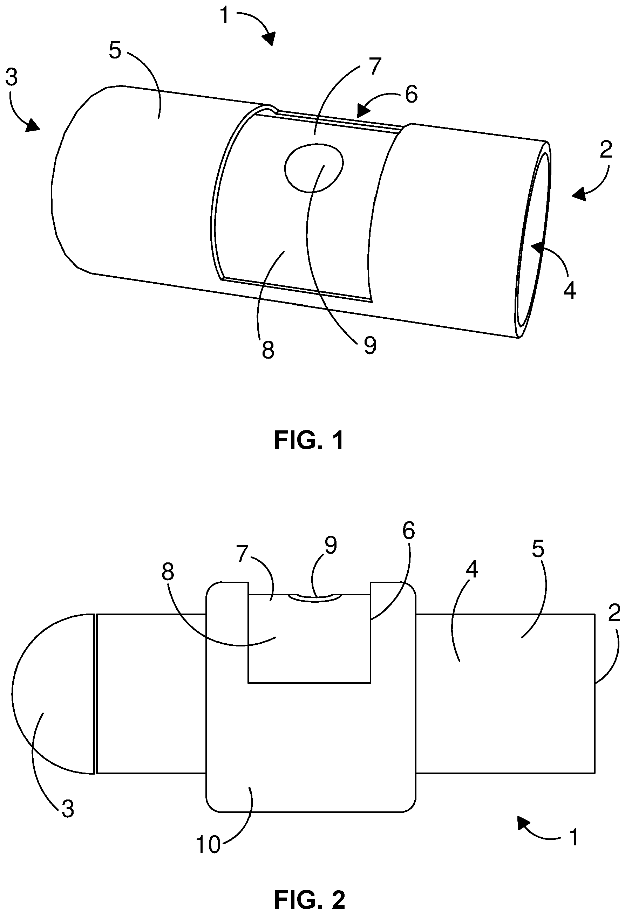

Figure 1 shows generally a catheter 1 according to the present invention. The catheter 1 is elongated in shape with a proximal end 2 (not shown) and a distal end 3. In this embodiment, the distal end 3 of the catheter 1 is flat in shape. Between the proximal end 2 and distal end 3 there is an internal conduit 4 which is largely blocked from view, in the figure 1, by the wall 5 of the catheter 1. The catheter 1 also comprises an opening 6. The catheter 1 also comprises an electrode 7. In this example, the electrode 7 is partially housed by the catheter 1, but comprises an exposed portion 8, which can be seen through the opening 6 through the wall 5 of the catheter 1. The electrode 7 comprises a port 9. In this example, the port 9 corresponds to the opening 6 of the catheter 1. In this example, the electrode 7 is an anode 7a. Also in this embodiment is a saline source (not shown) with an outlet (not shown) within the conduit 4 of the catheter 1.

Figure 2 shows generally part of a catheter 1 according to the present invention. The catheter 1 is elongated in shape with a proximal end 2 and a distal end 3. In this embodiment, the distal end 3 is slightly pointed with a rounded tip to aid insertion into position within the human body. In this embodiment, the distal end comprises an atraumatic shaped end 3. Between the proximal end 2 and distal end 3 there is an internal conduit 4 which is blocked from view by the wall 5 of the catheter 1. The catheter 1 also comprises an opening 6. The catheter 1 also comprises an electrode 7. In this example the electrode 7 is partially housed by the catheter 1, but comprises an exposed portion 8 which can be seen through the opening 6 through the wall 5 of the catheter. The electrode 7 comprises a port 9. In this example, the port 9 corresponds in positioning, at least partially, to the opening 6 of the catheter 1. The catheter 1 in this example comprises raised wall portions 10 that have a raised portion radially from the longitudinal axis of the catheter 1. The raised portion 10 protrudes radially from the axis of the catheter 1, beyond the wall 5 of the shaft of the catheter 1. In other examples, the catheter 1 may comprise a different number of raised portions, or different shaped or configured raised portions or may not comprise any raised portions. In this example, the raised wall portions 10 are 360 degrees around the circumference of the catheter 1 across the longitudinal direction of the catheter 1. Also in this example the raised wall portions 10 are 4 millimetres in length, along the longitudinal direction of the catheter 1, but different embodiments may have different sizes. In this example, the electrode 7 is a cathode 7c, but in alternative embodiments, the electrode could be an anode 7a, or indeed there may be a plurality of different electrodes 7. In this example, the wall 5 of the shaft of the catheter comprises a polymer. In this embodiment, the electrodes comprise platinum iridium or stainless steel. In this example, the electrode 7 protrudes in distance beyond the surface of the wall 5 of the shaft of the catheter 1 by 1 millimetre from the wall 5 of the shaft of the catheter 1. In alternative embodiments, the electrode 7 may protrude radially, from the axis of the catheter 1, beyond the wall 5 of the shaft of the catheter by a different distance, or indeed may be flush with the wall 5 of the shaft of the catheter 1.

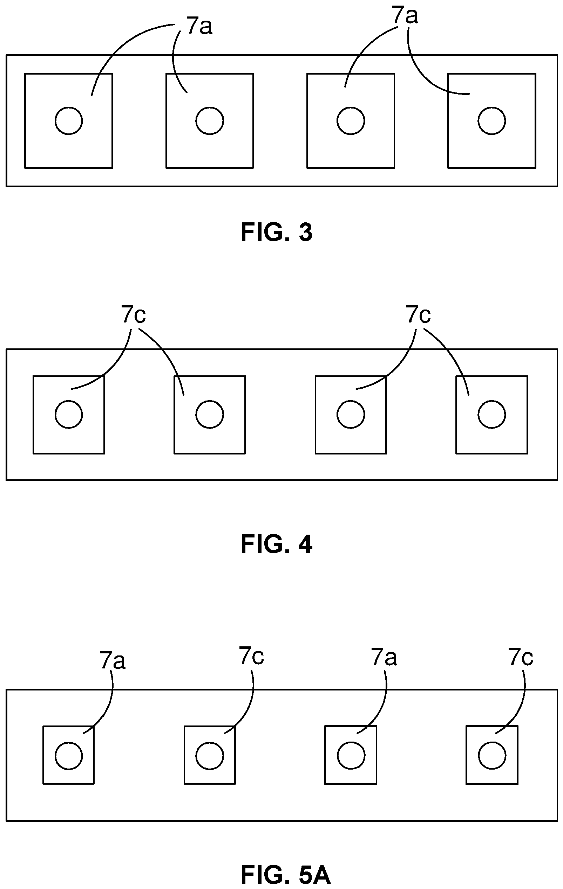

Figure 3 shows part of a catheter 1 with a plurality of electrodes 7. In this example, all of the electrodes 7 are anodes 7a.

Figure 4 shows part of a catheter with a plurality of electrodes 7. In this example, all of the electrodes 7 are cathodes 7c.

Figure 5A shows part of a catheter with a plurality of electrodes 7. In this example, the electrodes 7 comprise both anodes 7a and cathodes 7c, wherein the electrodes 7 alternate between anode 7a and cathode 7c electrodes 7. In alternative embodiments a different number of electrodes may be used.

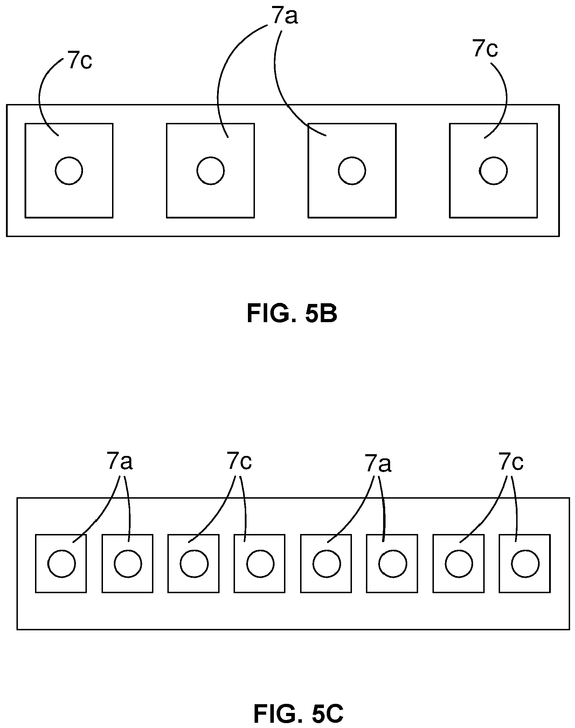

Figure 5B shows part of a catheter with four electrodes 7, with a pair of anode electrodes 7a in the middle and cathode electrodes 7c on either side of the anode electrodes 7a. In alternative embodiments, a different number of electrodes may be used.

Figure 5C shows part of a catheter with a plurality of electrodes 7 arranged in pairs of anodes 7a and pairs of cathodes 7c. The electrodes 7 alternate in pairs. In alternative embodiments, a different number of electrodes may be used.

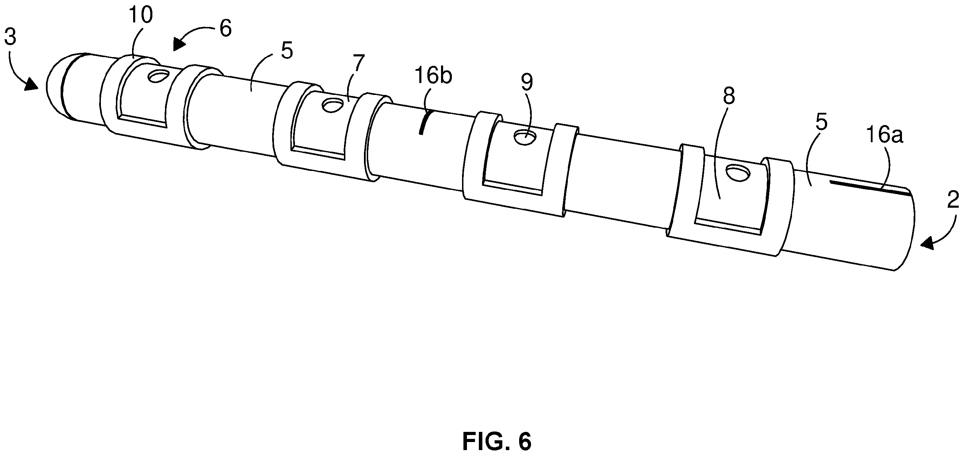

Figure 6 shows another catheter 1 according to the present invention. In this embodiment, there are 4 electrodes 7, all anodes 7a. The distal end 3 is pointed or round in shape to aid insertion into the human body and to position in the region of the heart. The four openings 6 of the catheter 1 are generally rectangular in shape and each opening 6 is 2.7 millimetre in length in the longitudinal direction of the catheter 1 and 2 millimetres in width across the longitudinal axis of the catheter. Alternative examples may have other sized and shaped openings. Alternative embodiments may have different size and shaped openings. Each opening 6 in this example, has a raised portion 10 surrounding the opening 6 and in this embodiment, the raised portion 10 extends around the catheter surrounding the opening 6. The raised portion 10 protrudes 1.2 millimetre from the wall 5 of the shaft of the catheter 1. Optionally, but in this embodiment, the raised portion 10 of the catheter enables insulation to be positioned inside the catheter in the internal areas within the catheter 1, adjacent to the raised portions 10 on the inside surface of the raised portions 10 of the catheter. The insulation (not shown) is configured that it provides insulation against the electric field but still enables any wires and cables to pass along the internal conduit 4 (not shown as within wall or walls 5) of the catheter 1. There is no insulation blocking the electrodes 7 at the openings 6, thus there is no insulation adjacent to the electrodes 7 on the surface of the electrode that corresponds to the corresponding opening 6. In this embodiment, the electrodes protrude through the openings 6 by 1 millimetre from, or beyond, the wall 5 of the shaft of the catheter 1, in a radial direction from the axis of the catheter 1. In this embodiment, the catheter 1 also comprises an orientation mark 16a towards the proximal end 2 of the catheter 1 on the wall 5 of the shaft of the catheter 1. The orientation mark 16a is a visible line indicating the direction that the electrodes 7 face. The orientation mark 16a is a line that runs partially along the longitudinal length of the wall 5 of the catheter. In this embodiment, the catheter also comprises an orientation mark 16b that is a radiopaque mark that is largely a semi-circular line that largely crosses the longitudinal direction of the catheter 1. The orientation mark 16b also indicates the electrode 7 facing direction, and with the aid of X-Rays or similar like radiation and imaging equipment, the electrode face orientation can be seen on a screen thus the desired positioning of the catheter 1 can be achieved. In the present example, the orientation mark 16b is shown towards the distal end of the catheter 1. However, in alternative embodiments that have an orientation mark 16b, the orientation mark may be positioned elsewhere on the catheter 1 for example, between the most distal electrode 7 and the distal end of the catheter 1.

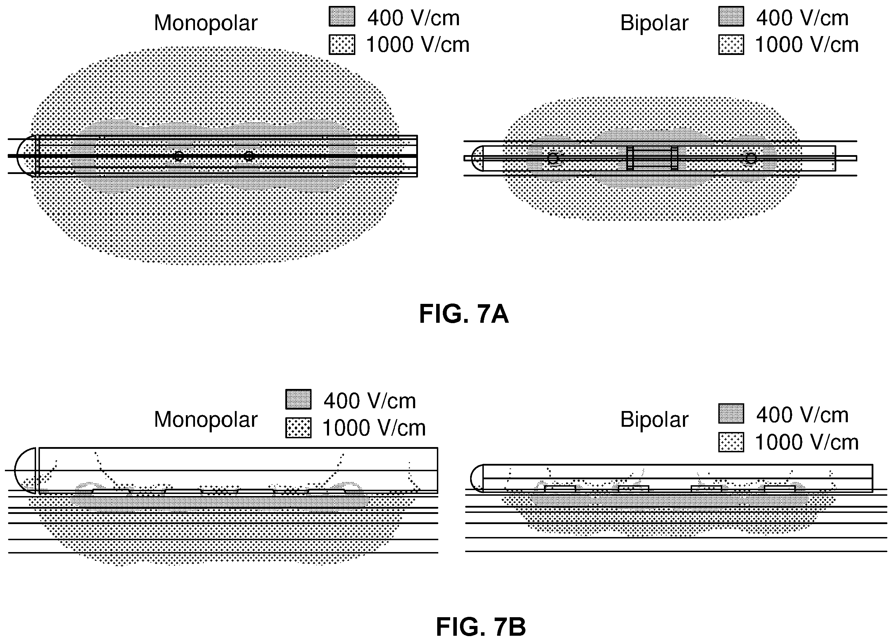

Figure 7A shows the electric field width for a monopolar arrangement and a bipolar arrangement, showing two field strength isoline 1000 volts per centimetre (V/cm) and 400 volts per centimetre (V/cm).

Figure 7B also shows the electric field depth for two field strength isoline 1000 volts per centimetre (V/cm) and 400 volts per centimetre (V/cm), for the same monopolar and bipolar arrangement as in figure 7A.

From this particular example the electric field strengths were:

| E-field V/cm | 400 | 400 | 400 | 1000 | 1000 | 1000 |
|---|---|---|---|---|---|---|
| Cases | X length mm | Y width mm | Z depth mm | X length mm | Y width mm | Z depth mm |
| Monopolar | 33.84 | 19.80 | 6.38 | 25.56 | 6.71 | 1.81 |
| Bipolar | 29.08 | 11.77 | 4.09 | 24.21 | 5.72 | 1.87 |

The monopolar gives a larger electric field area both in width from the electrode and depth from the electrode both for the 1000 volts/ centimetre field strength and the lesser field strength of 400 volts per centimetre.

Both examples of 7A and 7B had a voltage output of 1000 volts. The electric field was a pulsed electric field and comprised 60 monophasic pulses of a duration of 100 microseconds. In these 7A and 7B examples, the electrodes comprise stainless steel.

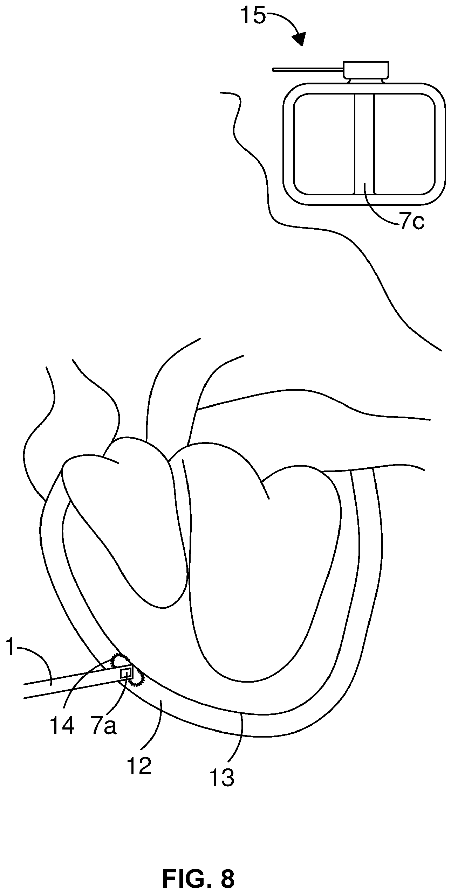

Figure 8 illustrates a system of the present invention where at least one electrode 7 is positioned via the chest wall of the patient to be positioned in the region of the heart of a patient, in this example in the pericardial space 12. In this example, the electrode 7 that is positioned within the body of the patient is an anode 7a. The system also comprises another oppositely charged electrode 7, a cathode 7c, that is positioned in use on the back of the patient so that in use the electric field is directed from the electrode 7a within the patient to the electrode 7c on the patients back. The electrode for use on the patients back comprises am off-the-shelf dispersive pad 15 approximately 180 by 115 millimetres and comprises a cathode electrode 7c. Within the body of the patient the opening of the electrode is positioned to face the epicardium 13 of the heart of the patient. Within the patient the opening of the electrode 7a is positioned to face the targeted cell bodies, in this example, targeted ganglionated plexi neuron cell bodies which are known to be giving wrong signals. The insulated layer of the catheter is positioned facing away from the epicardial tissue 13, in order to limit the electric field in non-targeted directions. In this embodiment 4 millilitres per minute of saline 14 is dispensed from the port of the electrode 7a of the catheter 1 before activation of the electrodes. The electrodes are activated. In this embodiment the voltage output comprises 1000 volts. The electric field comprises a pulsed electric field and comprises 75 monophasic pulses of a duration of 100 microseconds each followed by a pulse interval of 0.2 seconds. In this embodiment, the electrodes comprise platinum iridium.

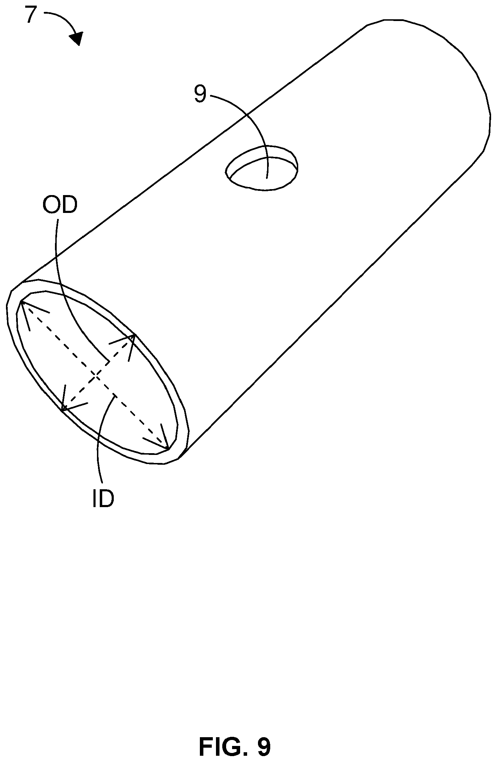

Figure 9 shows an electrode 7 suitable for use to position within a catheter 1 of the present invention. The electrode 7 comprises a port 9. In this embodiment the electrode 7 comprises a hollow cylindrical shape with an inner diameter (ID) of 1.9 mm and an outer diameter (OD) of 2.1 mm.

## Claims

1. A device, for ablating ganglionated plexi neuron cell bodies to treat cardiac disorders, the device comprising:
- at least one pair of oppositely charged electrodes;
- a source of electrical energy; and
- a catheter, the catheter comprising a proximal end, a distal end and an internal conduit between the proximal end and distal end, and the catheter further comprising at least one opening, and wherein at least one of the oppositely charged electrodes is at least partially housed in the catheter.

2. A device according to claim 1, wherein the at least one opening of the catheter is less than 180° radially around the circumference across the longitudinal axis of the catheter.

3. A device according to any preceding claim, wherein the electrical energy source comprises an output voltage in the range of 500 to 1500 Volts.

4. A device according to any preceding claim, wherein the electrical energy source output comprises a pulsed electric field.

5. A device according to any preceding claim, wherein the pulsed electric field has an output electric field strength greater than 1000 V/cm.

6. A device according to claim 4, wherein the pulsed electric field comprises monophasic pulses; or biphasic pulses.

7. A device according to claim 6, wherein the pulsed electric field comprises at least 60 repeat monophasic pulses per treatment site having a pulse duration of 100 µs.

8. A device according to any one of claims 4-7, wherein the pulse frequency is in the range of 0.1 to 10 Hz.

9. A device according to any one of claims 4 to 8, wherein the pulse frequency is synchronised with the Ventricular Refractory period of the heart of the patient.

10. A device according to any preceding claim, wherein each electrode comprises at least one port for fluid dispensing.

11. A device according to claim 10, wherein the at least one port of the electrode is positioned to correspond with the opening of the catheter.

12. A device according to any preceding claim, further comprising an insulating layer, wherein the insulating layer is fixed at least partially over the back of the at least one electrode such that the electric field is directed towards the front of the at least one electrode.

13. A device according to any preceding claim wherein at least one electrode of the oppositely charged electrodes that is at least partially housed in the catheter, protrudes through the at least one opening, external of the catheter, beyond the wall of the shaft of the catheter.

14. A system for ablating ganglionated plexi neuron cell bodies to treat cardiac disorders, the system comprising:
- at least one pair of oppositely charged electrodes;
- a source of pulsed electrical energy; and
- a catheter, the catheter comprising a proximal end, a distal end and an internal conduit between the proximal end and distal end, and the catheter further comprising at least one opening, and wherein at least one of the oppositely charged electrodes is at least partially housed in the catheter.

15. A system as claimed in claim 14 wherein the system further comprises a source of saline solution.
